# EUROPÄISCHE PATENTSCHRIFT

(11) **EP 0 324 447 B1**
(45) Veröffentlichungstag und Bekanntmachung des Hinweises auf die Patenterteilung: **11.09.1996**
(21) Anmeldenummer: 89100402.0
(22) Anmeldetag: 11.01.1989
(51) Int. Cl.: C12N 15/25, C07K 14/545

(54) **Mittel und Verfahren zur Herstellung eines rekombinanten, menschlichen Interleukin-1 alpha-Polypeptides**
Means and process for producing a recombinant human interleukin-1 alpha polypeptide
Moyens et procedes pour la production d'un polypetide de l'interleukin-1 alpha humaine recombinante

(30) Priorität: 15.01.1988 US 144457
(43) Veröffentlichungstag der Anmeldung: 19.07.1989
(73) Patentinhaber: F. HOFFMANN-LA ROCHE AG, 4002 Basel (CH)
(72) Erfinder: Lomedico, Peter T., Upper Montclair, NJ 07043 (US)
(74) Vertreter: Lederer, Franz, Dr.

(56) Entgegenhaltungen:
- EP-A- 0 188 920
- EP-A- 0 200 986
- EP-A- 0 259 160
- GENE, Band 49, Nr. 1 1986, Elsevier Science Publishers B.V., Amsterdam (NL); S.M. ZURAWSKI et al., Seiten 61-68
- PROCEEDINGS OF THE NATL. ACADEMY OF SCIENCES USA, Band 84, Juli 1987, Washington, DC (US); B. MOSLEY et al., Seiten 4572-4576

## Beschreibung

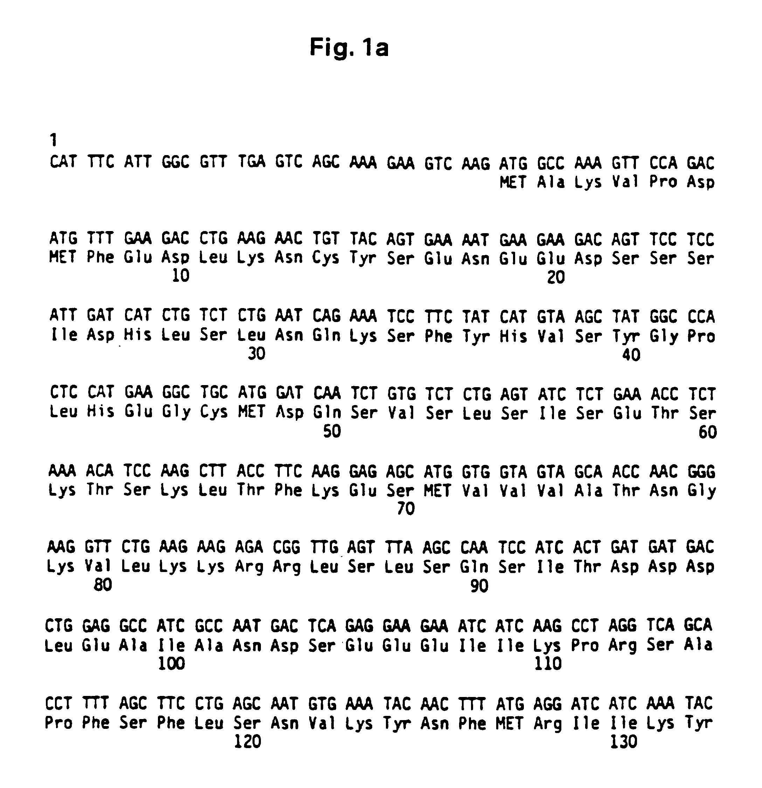

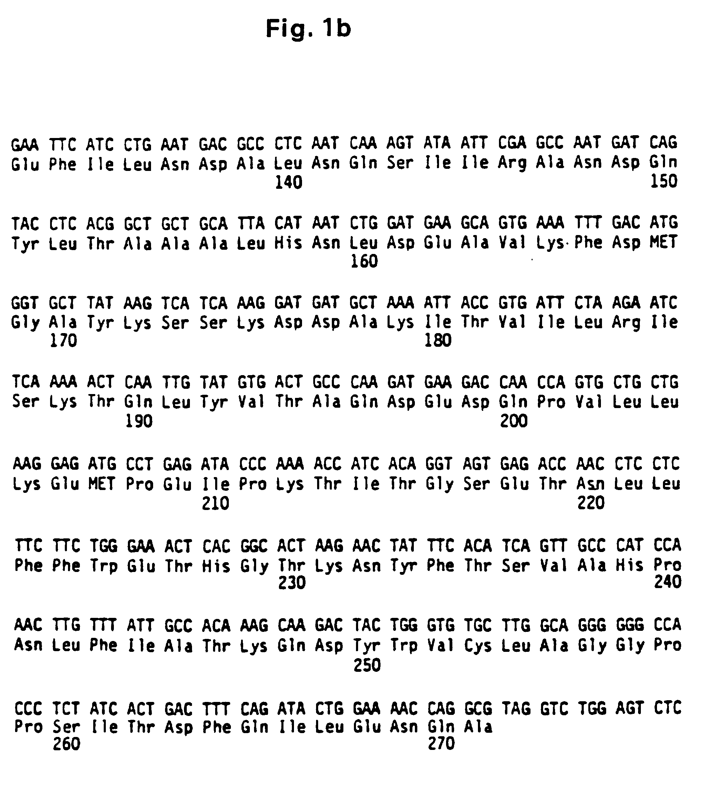

Zwei Formen von menschlichem Interleukin-1 sind bekannt, die sogenannte α- und die β-Form (March et al., Nature 315, 641-647 [1985]). Eine Analyse der Nukleotidsequenz einer DNA, die menschliches Interleukin-1α (hIL-1α) kodiert, zeigt einen grossen offenen Leseraster von 271 Aminosäuren, der dem hIL-1α Vorläuferpolypeptid mit einem Molekulargewicht von 30′606 Dalton entspricht. Die Nukleotidsequenz und die Aminosäuresequenz des hIL-1α Vorläuferpolypeptids, wie sie von Gubler et al., J. Immunol. 163, 2492-2497 (1986) bestimmt worden ist, ist in Figur 1a/1b gezeigt.

Die Europäische Patentanmeldung, Publikationsnummer 188 864, offenbart, dass der C-terminale Teil des hIL-1α Vorläuferpolypeptids, der sich vom Ser an Position 113 bis zum Ala an Posion 271 (Fig. 1a/1b) erstreckt, biologisch aktiv ist. Die Europäische Patentanmeldung, Publikationsnummer 188 920, offenbart hIL-1α Vorläuferpolypeptide, die sich in der Aminosäuresequenz von der Aminosäuresequenz wie sie in Figur 1a/1b gezeigt ist, dadurch unterscheiden, dass sie ein Ser an Position 114 haben anstelle eines Ala, wobei das Ser kodiert ist durch das Codon TCA. Die Europäische Patentanmeldung Nr. 188 920 offenbart ferner die Herstellung von verschiedenen hIL-1α Polypeptiden, nämlich den hIL-1α Polypeptiden mit der Aminosäuresequenz von Position 63-271, 113-271, 115-271, 123-271, 127-271, 128-271, 129-271, 113-267, 113-264, 113-266, 113-265 oder 128-267 der in Figur 1a/1b gezeigten Aminosäuresequenz, wobei, wie bereits oben erwähnt, der Alaninrest an Position 114, sofern vorhanden, durch ein Serinrest ersetzt worden ist. Die Europäische Patentanmeldung, Publikationsnummer 200 986 beschreibt die Herstellung eines rekombinanten hIL-1α Polypeptides mit einer Aminosäuresequenz die sich von Position 118 bis Position 271 der in Figur 1a/1b gezeigten Aminosäuresequenz erstreckt und einen Methioninrest an Position 117 enthält. Dieser Methioninrest is durch das ATG-Codon kodiert, das zur Expression des DNA-Fragmentes, das die Aminosäuresequenz von Position 118-271 kodiert, verwendet wird. Da das natürliche hIL-1α Polypeptid einen Serinrest an Position 117 aufweist, ist das rekombinante hIL-1α Polypeptid, wie es gemäss der Europäischen Patentanmeldung, Publikationsnummer 200 986 hergestellt worden ist, bezüglich des N-Terminus verschieden von einem entsprechenden natürlichen reifen hIL-1α Polypeptid, wie es aus einer menschlichen Körperflüssigkeit oder aus einem Ueberstand von kultivierten menschlichen Zellen isoliert werden kann. Die Anwesenheit des modifizierten N-Terminus im rekombinanten hIL-1α Polypeptid könnte Nebeneffekte in einem Patienten, der mit dem rekombinanten Polypeptid behandelt wird, zur Folge haben. Um solche Nebeneffekte zu vermeiden, wurden Wege gesucht um Methoden zu finden um rekombinante reife hIL-1α Polypeptide mit einer Aminosäuresequenz die identisch ist mit der Aminosäuresequenz von natürlichem, reifem hIL-1α, herzustellen. Es wurde gefunden, dass überraschenderweise das rekombinante, reife hIL-1α Polypeptid mit der Aminosäuresequenz 
Ser Phe Leu Ser Asn Val Lys Tyr Asn Phe MET Arg Ile Ile Lys Tyr
Glu Phe Ile Leu Asn Asp Ala Leu Asn Gln Ser Ile Ile Arg Ala Asn
Asp Gln Tyr Leu Thr Ala Ala Ala Leu His Asn Leu Asp Glu Ala Val
Lys Phe Asp MET Gly Ala Tyr Lys Ser Ser Lys Asp Asp Ala Lys Ile
Thr Val Ile Leu Arg Ile Ser Lys Thr Gln Leu Tyr Val Thr Ala Gln
Asp Glu Asp Gln Pro Val Leu Leu Lys Glu MET Pro Glu Ile Pro Lys
Thr Ile Thr Gly Ser Glu Thr Asn Leu Leu Phe Phe Trp Glu Thr His
Gly Thr Lys Asn Tyr Phe Thr Ser Val Ala His Pro Asn Leu Phe Ile
Ala Thr Lys Gln Asp Tyr Trp Val Cys Leu Ala Gly Gly Pro Pro Ser
Ile Thr Asp Phe Gln Ile Leu Glu Asn Gln Ala, (I)
 wobei diese Aminosäuresequenz der Sequenz von Position 117 bis Position 271, der in Figur 1a/1b gezeigten Aminosäuresequenz entspricht, in einem einzelligen Organismus ohne den N-terminalen Methioninrest hergestellt werden kann.

Die vorliegende Erfindung liefert ein verfahren zur Herstellung eines homogenen, rekombinanten, menschlichen Interleukin-1α Polypeptides mit der Aminosäuresequenz (I), DNAs, die für ein solches Polypeptid kodieren, rekombinante Vektoren, die eine DNA, die für das Polypeptid kodiert, enthalten und einzellige Organismen, die einen solchen rekombinanten Vektor enthalten, wobei diese einzelligen Organismen fähig sind die oben genannte DNA zu exprimieren.

Die Polypeptide der vorliegenden Erfindung können mittels konventioneller Methoden der Peptidsynthese, in flüssiger oder, vorzugsweise an fester Phase, z.B. gemäss der Methode von Merrifield (J. Am. Chem. Soc. 85, 2149-2154 [1963]), oder mittels anderer, gleichwertiger Methoden des Standes der Technik, hergestellt werden.

Andererseits und vorzugsweise werden die erfindungsgemässen Polypeptide unter Verwendung von Methoden der rekombinanten DNA-Technologie hergestellt. Dabei wird ein einzelliger Organismus, der einen rekombinanten Vektor enthält, der eine DNA enthält, die für ein erfindungsgemässes reifes menschliches Interleukin-lα Polypeptid kodiert, unter Bedingungen kultiviert, die geeignet sind, die Expression der oben genannten DNA zu ermöglichen und das durch den einzelligen Organismus hergestellte menschliche Interleukin--1α Polypeptid aus der Kultur isoliert.

Solch einzellige Organismen können prokaryotische oder eukaryotische Zellen sein. Eine grosse Zahl solcher einzelligen Organismen sind kommerziell erhältlich oder, frei verfügbar, bei Hinterlegungsstellen wie z.B. der American Type Culture Collection (ATCC), 12301 Parklawn Drive, Rockville, Maryland, USA, hinterlegt. Beispiele von prokaryotischen Zellen sind Bakterien wie E.coli [z.B. E.coli M15, von Villarejo et al. in J. Bacteriol. 120, 466-474 [1974] als DZ291 beschrieben, E.coli 294 (ATCC-Nr. 31446), E.coli RR1 (ATCC-Nr. 31343) oder E.coli W3110 (ATCC-Nr. 27325)], Bacilli wie z.B. B.subtilis oder Enterobacteriaceae wie z.B. Salmonella typhimurium und Serratia marcescens. Besonders bevorzugt ist E.coli Stamm MC1061 enthaltend das Plasmid pRK248cIts (siehe Beispiel). Andererseits können Hefezellen.

wie z.B. Saccharomyces cerevisiae als Wirtsorganismen verwendet werden. Eine grosse Anzahl eukaryotischer Zellen, die als Wirtsorganismen für die Expression rekombinanter Vektoren geeignet sind, sind dem Fachmann bekannt. Säugerzellen, wie z.B. CV-1 (ATCC-Nr. CCL 70) und Abkömmlinge davon, z.B. COS-1 (ATCC-Nr. CRL 1650) oder COS-7 (ATCC-Nr. CRL 1651) werden bevorzugt verwendet. Auch ist es möglich, Insektenzellen, wie sie z.B. von Smith et al. (Mol. Cell. Biol. 2, 2156-2165 [1983]) beschrieben worden sind, zu verwenden.

Verschiedene Methoden zur Einführung von rekombinanten Vektoren in einzellige Organismen sind bekannt. Beispiele für solche Methoden sind Mikroinjektion, Transfektion oder Transduktion. Der Fachmann hat keine Schwierigkeiten die für ein bestimmtes Wirtsorganismus am besten geeignete Methode auszuwählen.

Die rekombinanten Vektoren, die in der vorliegenden Erfindung verwendet werden, sind Vektoren, die eine DNA enthalten, die für ein erfindungsgemässes hIL-1α Polypeptid kodieren, wie die DNAs mit der Nukleotidsequenz von Position 385 bis Position 852 oder von Position 394 bis Position 852 der in Figure 1a/1b gezeigten Nukleotidsequenz. Solche DNAs können mittels konventioneller chemischer Methoden synthetisiert werden, z.B. mittels der Phosphotriestermethode, wie sie von Narang et al. in Meth. Enzymol. 68, 90-108 [1979] beschrieben ist, oder mittels der Phosphodiestermethode von Brown et al. (Meth. Enzymol. 68, 109-151 [1979]). Bei beiden Methoden werden zuerst längere Oligonukleotide synthetisiert und anschliessend in vorgegebener Art und Weise miteinander verbunden. Die Nukleotidsequenz der DNA kann identisch sein zur Nukleotidsequenz wid sie oben angegeben ist oder kann teilweise oder vollständig verschieden davon sein. Dies ist darin begründet, dass der genetische Code degeneriert ist. Das bedeutet, dass eine Aminosäure durch verschiedene Codons kodiert wird. Die gewählten Codons können dem bevorzugten Codonverwendungsschema des Wirtsorganismus angepasst sein (Grosjean et al., Gene 18, 199-209 [1982]). Es muss darauf geachtet werden, dass die DNA, die auf diese Art und Weise erhalten wird, nicht Teilsequenzen enthält, die die Konstruktion des rekombinanten Vektors erschweren, z.B. durch Einführung einer unerwünschten Restriktionsenzymschnittstelle, oder durch Hemmung der Expression des Polypeptides.

Die DNA, die für ein erfindungsgemässes hIL-1α Polypeptid kodiert, kann auch hergestellt werden, indem eine DNA, die für das hIL-1α Vorläuferpolypeptid kodiert, aus einer menschlichen cDNA-Bibliothek oder aus einer genomischen Bibliothek, gemäss Verfahren wie sie dem Fachmann bekannt sind, isoliert wird (z.B. March et al., supra; Gubler et al., supra) und dann unter Verwendung von Restriktionsendonukleasen und möglicherweise kurzen synthetischen Oligonukleotiden gemäss Verfahren wie sie in der rekombinanten DNA-Technologie bekannt sind in die gewünschte Grösse und Nukleotidsequenz überführt wird.

Eine grosse Anzahl von Vektoren, z.B. diejenigen, die in der Europäischen Patentanmeldung mit der Publikationsnummer 200 986 erwähnt sind, können zur Herstellung der erfindungsgemässen rekombinanten Vektoren verwendet werden. Diese Vektoren enthalten Elemente die für die Transkription und Translation der DNA, die für ein erfindungsgemässes hIL-1α Polypeptid kodiert benötigt werden, sowie Elemente die der Aufrechterhaltung und Replikation des Vektors im Wirtsorganismus dienen. Die Auswahl eines geeigneten Vektors für die Konstruktion der rekombinanten Vektoren der vorliegenden Erfindung und die Selektion eines geeigneten einzelligen Wirtsorganismus, der mit diesem rekombinanten Vektor kompatibel ist, ist für den Fachmann ohne erfinderischen Beitrag möglich.

Andererseits und vorzugsweise werden die erfindungsgemässen rekombinanten Vektoren durch Modifizierung von rekombinanten Vektoren, die eine DNA enthalten, die für ein bekanntes hIL-1α Polypeptid kodiert, unter Verwendung von Oligonukleotiden durch gezielte Mutagenese (oligonucleotide directed site specific mutagenesis) gemäss Morinaga et al., BIO/TECHNOLOGY 2, 636-639 (1984), hergestellt. Das Plasmid phil #1-154* ist ein Beispiel für einen solchen rekombinanten Vektor, der eine DNA enthält, die für ein bekanntes hIL-1α Polypeptid kodiert. Die Konstruktion dieses Plasmids ist in der Europäischen Patentanmeldung mit der Publikationsnummer 200 986 ausführlich beschrieben.

Die Art und Wiese, wie die Expression der erfindungsgemässen Polypeptide erfolgt, ist abhängig vom verwendeten Expressionsvektor und vom Wirtsorganismus. Ueblicherweise werden die Wirtsorganismen, die den Expressionsvektor enthalten, unter Bedingungen, die für das Wachstum der Wirtsorganismen optimal sind, vermehrt. Gegen Ende des exponentiellen Wachstums, wenn die Zunahme der Zellzahl pro Zeiteinheit abnimmt, wird die Expression des Polypeptids induziert, d.h., die das Polypeptid kodierende DNA transkribiert und die transkribierte mRNA translatiert. Die Induktion kann durch Zugabe eines Induktors oder eines Derepressors zum Wachstumsmedium oder durch Veränderung eines physikalischen Parameters, z.B. durch eine Temperaturänderung, erfolgen.

Das in den Wirtsorganismen produzierte Polypeptid kann mittels spezieller Transportmechanismen aus der Zelle sezerniert, oder durch Aufbrechen der Zelle isoliert werden. Die Zellen können mechanisch (Charm et al., Meth. Enzymol. 22, 476-556 [1971]), enzymatisch (Lysozymbehandlung) und/oder chemisch (Detergenzbehandlung, Harnstoff- oder Guanidin•HCl-Behandlung, etc.) aufgebrochen werden.

In Eukaryonten werden Polypeptide, die von der Zelle sezerniert werden, in Form eines Vorläufermoleküls synthetisiert. Durch Abspaltung des sogenannten Signalpeptids entsteht das reife Polypeptid. Da prokaryotische Wirtsorganismen nicht fähig sind eukaryotische Signalpeptide von Vorläufermolekülen abzuspalten, müssen eukaryotische Polypeptide in prokaryotischen Wirtsorganismen direkt in ihrer reifen Form exprimiert werden. Das Translationsstartsignal AUG, dem auf dem Niveau der DNA das Codon ATG entspricht, bewirkt, dass alle Polypeptide in einem prokaryotischen Wirtsorganismus mit einem Methioninrest am N-Terminus synthetisiert werden. In gewissen Expressionssystemen wird dieser N-terminale Methioninrest abgespalten.

Die erfindungsgemässen Polypeptide können mittels bekannter Methoden bis zur Homogenität gereinigt werden, wie z.B. mittels Zentrifugation bei unterschiedlichen Geschwindigkeiten, mittels Präzipitation mit Ammoniumsulfat, mittels Dialyse (bei Normaldruck oder bei reduziertem Druck), mittels präparativer Isoelektrofokussierung, mittels präparativer Gelelektrophorese oder mittels verschiedener chromatographischer Methoden wie Gelfiltration, Hochleistungsflüssigchromatographie (HPLC), Ionenaustauschchromatographie, Umkehrphasenchromatographie und Affinitätschromatographie (z.B. an Sepharose™ Blau CL-6B oder an trägergebundenen monoklonalen Antikörpern die gegen ein hIL-1α Polypeptid gerichtet sind).

Die erfindungsgemässen homogenen rekombinanten hIL-1α Polypeptide können auf bekannte Art und Weise verwendet werden z.B. als Vakzinadjuvans oder um das Immunsystem eines Patienten zu stimulieren, um die Abwehr gegen pathogene Agentien zu verbessern, oder um die körpereigenen Abwehr gegen neoplastische Krankheiten zu unterstützen. Andere bekannte klinische Verwendungszwecke für hIL-1α betreffen die Förderung der Wundheilung durch Stimulation der Fibroblastenproliferation und die Förderung der Genesung von schwer kranken, proteinunterernährten Patienten.

Homogene hIL-1α Polypeptide, die gemäss der vorliegenden Erfindung hergestellt werden, können an Säuger zur Behandlung der oben angegebenen klinischen Indikationen verabreicht werden. Die Verabreichung kann gemäss beliebigen konventionellen Methoden, z.B. durch parenterale Applikation, entweder intravenös, subcutan oder intramuskulär, erfolgen. Die benötigte Dosis variiert mit der zu behandelnden Indikation, der Ernsthaftigkeit des Falles, der Dauer der Behandlung und der Art und Weise der Verabreichung. Eine geeignete pharmazeutische Verabreichungsform kann erhalten werden indem steril filtriertes, lyophilisiertes hIL-1α Polypeptid vor Gebrauch auf übliche Art und Weise rekonstituiert wird. Es ist für den Fachmann ohne weiteres möglich pharmazeutische Zusammensetzungen, die ein erfindungsgemässes homogenes, menschliches Interleukin-1α enthalten, herzustellen. Dabei wird ein erfindungsgemässes Interleukin-1α mit kompatiblen, pharmazeutisch verträglichen Trägermaterialien, wie z.B. Puffern, Stabilisatoren, Bakteriostatika oder anderen Zusätzen, die üblicherweise in parenteralen Dosierungsformen verwendet werden, kombiniert.

### Beispiel

Die Methode der gezielten Mutagenese, wie sie von Morinaga et al. in BIO/TECHNOLOGY 2, 636-639 (1984) beschrieben ist, wurde verwendet, um das Plasmid phil #1-154* (Europäische Patentanmeldung, Publikationsnummer 200 986) so zu verändern, dass Plasmide entstehen, die Gene enthalten, die für die erfindungsgemässen hIL-1α Polypeptide kodieren. Als erstes wurden Oligonukleotide folgender Nukleotidsequenz hergestellt: GGA ATT AAT ATG AGC TTC CTG AGC (O. 117), GGA ATT AAT ATG CTG AGC AAT GTG (O. 119) und

GGA ATT AAT ATG AGC AAT GTG AAA (O. 120).

Diese Oligonukleotide wurden mittels der Phosphoramiditfestphasenmethode von Matteucci et al., J. Am. Chem. Soc. 103, 3185-3191 (1981), hergestellt.

Das Plasmid phil #1-154* wurde unter Verwendung von BglII und SalI geschnitten, bzw. unter Verwendung von PstI linearisiert. In drei getrennten Reaktionen, wurden die Oligonukleotide angelagert, in eine doppelsträngige Form überführt, ligiert und in E.coli K-12 Stamm MC1061 (Casadaban et al., J. Mol. Biol. 138, 179-207 [1980]), enthaltend das Plasmid pRK248cIts (Bernard et al., Meth. Enzym. 68, 482-492 [1979]), unter Verwendung der CaCl₂-Methode (Maniatis et al., in "Molecular Cloning: A Laboratory Manual", Seiten 250-251, Cold Spring Harbor Laboratory [1982]), transformiert. E.coli MC1061 und das Plasmid pRK248cIts sind bei der American Type Culture Collection, 12301 Parklawn Drive, Rockville, Maryland, USA unter den Hinterlegungsnummern ATCC-Nr. 53338 bzw. ATCC-Nr. 33766 erhältlich.

Kolonien, die Plasmide mit der gewünschten Mutation enthalten, wurden mittels Hybridisierung (Maniatis et al., supra, Seiten 312-315) identifiziert. Dabei dienten die Oligonukleotide (O. 117), (O. 119) und (O. 120), die zuerst mit γ-³²P-ATP am 5' Ende, unter Verwendung von Polynukleotidkinase, gemäss dem Verfahren von Maniatis et al, supra, Seite 396, markiert worden waren, als Proben.

Oligonukleotide (O. 117) führte zur Ueberführung von phil #1-154* in pHuIL-1α (117-271) mit der folgenden Sequenzmodifikation:

kodiert für
zusätzliche Aminosäuren
118 119 120 121
phil #1-154* ATGAATAGAATTCGGATCCGC TTC CTG AGC AAT
Met Phe Leu Ser Asn
117 118 119 120
pHuIL-1α (117-271) GGAATTAATATG AGC TTC CTG AGC
Met Ser Phe Leu Ser

Auf ähnliche Art und Weise entstand bei der Verwendung des Oligonukleotids (O. 119) das Plasmid pHuIL-1α (119-271) mit folgender Sequenz: 
119 120 121 122
GGAATTAATATG CTG AGC AAT GTG
Met Leu Ser Asn Val;
 und bei der Verwendung des Oligonukleotids (O. 120) das Plasmid pHuIL-1α (120-271) mit folgender Sequenz: 
120 121 122 123
GGAATTAATATG AGC AAT GTG AAA
Met Ser Asn Val Lys.
 DNA-Sequenzanalyse bestätigte die oben gezeigten Mutationen.

E.coli MC1061/pRK248cIts enthaltend phil #1-154 (siehe Europäische Patentanmeldung, Publikationsnummer 200 986; das Plasmid ist fähig hIL-1α (118-271) zu exprimieren), pHuIL-1α (117-271), pHuIL-1α (119-271) oder pHuIL-1α (120-271) wurden in M9 Medium (maniatis et al., supra, Seiten 68-69) enthaltend Ampicillin bei 30°C wachsen gelassen, bis die Absorption bei 550 nm (A₅₅₀) einen Wert von 0,7 erreichte. Danach wurden die Kulturen während 3 Stunden bei 42°C inkubiert. Die Bakterienzellen aus 1 ml Kultur wurden mittels Zentrifugation gesammelt und in 50 µl 7 M Guanidin•HCl solubilisiert. Der Rohextrakt wurde unter Verwendung des Mausthymocytenpcolifecationstests (Mizel et al., J. Immunol. 120, 1497-1508 [1978]) auf Interleukin-l Bioaktivität geprüft. Alle vier Extrakte zeigten Interleukin-1-Aktivität.

Unlösliche cytoplasmatische Einschlusskörper wurden aus allen vier induzierten Kulturen isoliert, wie üblich gereinigt, solubilisiert und mit einer SDS Polyacrylamidgelelektrophorese (Laemmli, Nature 227, 680-685 [1970]) aufgetrennt. Die Interleukin-la-Banden wurden jeweils mittels der Methode von Aebersold et al., J. Biol. Chem. 261, 4229-4238 (1986), aus dem Gel eluiert und einer N-terminalen Aminosäuresequenzanalyse gemäss Hewick et al., J. Biol. Chem. 256, 7990-7997 (1981) unterworfen. Folgende Resultate wurden erhalten:

### hIL-1α (117-271):

Ser Phe Leu Ser Asn Val Lys Tyr Asn Phe

### hIL-1α (118-271):

Met Phe Leu Ser Asn Val Lys Tyr Asn Phe

### hIL-1α (119-271):

Met Leu Ser Asn Val Lys Tyr Asn Phe Met

### hIL-1α (120-271):

Ser Asn Val Lys Tyr Asn Phe Met Arg Ile

Es ist sofort ersichtlich, dass der endterminale Methioninrest beim hIL-1α (117-271) und beim hIL-1a (120-271) abgespalten wurde, nicht aber beim hIL-1α (119-271) und beim hIL-1α (118-271).

Die hIL-1α Polypeptide hIL-1α (117-271), hIL-1α (118-271) und hIL-1α (120-271) wurden aus Ueberständen von Lysaten, von mit den Plasmiden pHuIL-1α (117-271), phil #1-154 bzw. pHuIL-1α (120-271) transformierten E.coli MC1061/pRK248cIts, wie folgt gereinigt:
1. Kulturen enthaltend 5 kg jeder Transformante wurden mit H₂SO₄ auf pH 1,8 eingestellt und während 30 Minuten bis 1 Stunde bei Raumtemperatur stehen gelassen. Die durch die Säurebehandlung getöteten Bakterien wurden mittels Zentrifugation gewonnen.
2. Das so erhaltene Bakterienzellsediment wurde in 18 1 Wasser resuspendiert und die Suspension während 1 Stunde bei Raumtemperatur mit einem mechanischen Scherrührer gerührt.
3. Das resuspendiert Material wurde durch zwei doppellagige Käsetücher filtriert. Dabei werden Zellbruchstücke entfernt.
4. Das filtrierte Material wurde zweimal, zuerst bei 15°C, dann bei etwa 30°C in einen Manton-Gaulin Zellhomogenisator (Meth. Enzymol. 22, 482-484 [1971]) bei einem Druck von 8,000 psi (entspricht etwa 5,5 x 10⁷ Pa), homogenisiert.
5. Das Homogenat wurde in einer Sorvallzentrifuge (Du Pont, Wilmington, Delaware, USA) bei 4°C während 1 Stunde bei 8,000 rpm, unter Verwendung eines GS-3 Rotors (Du Pont) zentrifugiert. Etwa 16,6 1 Ueberstand wurde gewonnen und das Sediment verworfen.
6. Der Ueberstand wurde durch einen 0,8/0,2 Micron Sartorius Filter (erhältlich von Sartorius Filters Inc., Hayward, California, USA oder von Sartorius-Membranfilter GmbH, D-3400 Göttingen), filtriert. Anschliessend wurde der pH mit 50%-iger (v-v) Natriumhydroxidlösung auf pH 3 eingestellt.
7. Der Ueberstand wurde auf eine mit 0.02 M Essigsäure äquilibrierte 10 x 40 cm Nugel P-SP Silica-Säule (ein Sulfopropylkationaustauscher; 40-60 micron, 200 Angstrom; Separation Industries, Metuchen, New Jersey, USA), aufgetragen. Nach dem Auftragen der Probe, wurde die Säule mit 31 l 0,02 M Essigsäure gewaschen und mit 30 mM Tris-HCl, pH 8,0 eluiert. Das Säuleneluat wurde spektrophotometrisch bei 280 nm auf die Anwesenheit von Proteinen überwacht. Zwei Proteinfraktionen wurden erhalten, nämlich eine 4,5 l Fraktion und eine 7,5 l Hauptfraktion.
8. Die Hauptfraktion aus Schritt 7 wurde auf eine mit 30 mM Tris-HCl, pH 8,0 äquilibrierte 10 x 40 cm NuGel P-DE Silicasäule (ein Diäthylaminoäthylanionenaustauscher; 40-60 Micron, 200 Angstrom; Separation Industries) aufgetragen. Die Säule wurde mit 17 l 30 mM Tris-HCl (pH 8,0), 0,075 M NaCl gewaschen und dann mit 13 l 30 mM Tris-HCl (pH 8,0), 0,25M NaCl eluiert. Das Eluat wurde spektrophotometrisch auf die Anwesenheit von Proteinen überwacht. Eine 13 l Proteinfraktion wurde so erhalten.
9. Das Eluat von Schritt 8 wurde vierfach mit 3,7 mM Essigsäure pH 4,8 verdünnt, auf pH 4,8 eingestellt und mit einer Fliessgeschwindigkeit von 150 ml/Minute auf eine 5 x 250 cm NuGel-P-SP HPLC Säule (Separation Industries) aufgetragen. Die Säule wurde dann mit einem linearen Gradienten von pH 4,8 (3,75 mM Essigsäure) bis pH 6,8 (10 mM K₂HPO₄) während 120 Minuten, bei einer Durchflussrate von 75 ml/Minute eluiert. Eine 9,3 l Proteinfraktion wurde auf diese Art und Weise erhalten.
10. Das Eluat der HPLC-Säule wurde unter Verwendung einer Amiconkonzentriervorrichtung (1,000 Molekulargewicht Ausschlussgrenze, erhältlich von Amicon, Div. von W.R. Grace & Co., Danvers, Massachusetts, USA) auf etwa 225 ml konzentriert und dann auf zwei in Serie geschaltete 10 x 100 cm Sephadex™ G-50 Säulen (Pharmacia Fine Chemicals,
   Piscataway, New Jersey, USA), die mit 50 mM K₂HPO₄ und 0.1M NaCl, pH 6,8 äquilibriert worden waren, aufgetragen. Eine 800 ml Proteinfraktion wurde erhalten. Diese wurde unter Verwendung einer Amicon Spiralpatrone (30,000 Molekulargewicht Ausschlussgrenze) depyrogenisiert. Das Endprodukt war in einem Volumen von etwa 1,070 ml.

Falls nicht anders erwähnt, wurden die oben genannten Reinigungsschritte bei 4°C durchgeführt, mit Ausnahme der HPLC-Säule die bei Raumtemperatur laufen gelassen wurde.

Die gereinigten hIL-1α (117-271), hIL-1α (118-271) und hIL-1α (120-271) Polypeptide wurden einer SDS-Polyacrylamidgelelektrophorese unterworfen. Das Gel wurde mit Coomassie blau gefärbt. Es wurde gefunden das die Proteine in homogener Form vorliegen.

Die Bioaktivität wurde bei den gereinigten Proteinen unter Verwendung des D10 Thymocytenproliferationstestes von Kaye et al., J. Exp. Med. 158, 836-856 (1983) durchgeführt. Folgende Resultate wurden erhalten.

**Tabelle I**

| Aktivität der hIL-1α Polypeptide | | |
|---|---|---|
| Protein | Lösliches hIL-1α im rohen Homogenat^{a} (mg/kg) | Spezifische Aktivität des gereinigten Proteins (Einheiten/mg) |
| hIL-1α (117-271) | 1,800 | 2 x 10⁹ |
| hIL-1α (118-271) | 600 | 2 x 10⁹ |
| hIL-1α (120-271) | 600 | 2 x 10⁹ |

| | | |
|---|---|---|
| ^{a} - Die Menge des löslichen hIL-1α im rohen Homogenat wurde basierend auf einem Immuntestverfahren unter Verwendung von hIL-1α spezifischen Antikörpern bestimmt. | | |

Die Daten in Tabelle I, zusammen mit den Daten wie sie in Tabelle I der Europäischen Patentanmeldung mit der Publikationsnummer 200 986 präsentiert worden sind, zeigen, dass die exakte Länge der hIL-1α Proteine nicht kritisch ist, solange die minimale C-terminale Sequenz, die sich von Position 132 bis Position 271, der in Figur 1a/1b gezeigten Aminosäuresequenz erstreckt, im Molekül vorhanden ist. Alle in Tabelle I aufgeführten Proteine haben in etwa die gleiche spezifische Bioaktivität. Die Daten zeigen auch, dass die Anwesenheit oder Abwesenheit eines N-terminalen Methioninrestes auf die Aktivität keinen Einfluss hat. Das Polypeptid hIL-1α (118-271), das einen zusätzlichen Methioninrest am N-Terminus besitzt, war genau so aktiv wie die anderen Proteine, die keinen N-terminalen Methioninrest haben.

Die Daten in Tabelle I zeigen auch, dass überraschenderweise hIL-1α (117-271) in etwa dreimal grösserer Menge exprimiert wird als das lösliche hIL-1α (118-271) oder das lösliche hIL-1α (120-271). Dies obwohl die Gene, die für diese Proteine kodieren, im gleichen Vektor und im gleichen Wirtsorganismus waren und obwohl die Zellen auf gleiche Art und Weise kultiviert wurden und auf gleiche Art und Weise geerntet wurden.

Die vorliegende Erfindung wird besser verständlich, wenn sie im Zusammenhang mit der beiliegenden Figur 1a/1b betrachtet wird, die die Nukleotidsequenz und die Aminosäuresequenz des humanen Interleukin-1α cDNA zeigt.

## Patentansprüche (Patentansprüche für folgende(n) Vertragsstaat(en): ; AT, BE, CH, DE, FR, GB, IT, LI, LU, NL, SE)

1. Eine DNA, die für ein menschliches Interleukin-1α Polypeptid der Aminosäuresequenz 
Ser Phe Leu Ser Asn Val Lys Tyr Asn Phe MET Arg Ile Ile Lys Tyr
Glu Phe Ile Leu Asn Asp Ala Leu Asn Gln Ser Ile Ile Arg Ala Asn
Asp Gln Tyr Leu Thr Ala Ala Ala Leu His Asn Leu Asp Glu Ala Val
Lys Phe Asp MET Gly Ala Tyr Lys Ser Ser Lys Asp Asp Ala Lys Ile
Thr Val Ile Leu Arg Ile Ser Lys Thr Gln Leu Tyr Val Thr Ala Gln
Asp Glu Asp Gln Pro Val Leu Leu Lys Glu MET Pro Glu Ile Pro Lys
Thr Ile Thr Gly Ser Glu Thr Asn Leu Leu Phe Phe Trp Glu Thr His
Gly Thr Lys Asn Tyr Phe Thr Ser Val Ala His Pro Asn Leu Phe Ile
Ala Thr Lys Gln Asp Tyr Trp Val Cys Leu Ala Gly Gly Pro Pro Ser
Ile Thr Asp Phe Gln Ile Leu Glu Asn Gln Ala
 kodiert.

2. Ein rekombinanter Vektor, der eine DNA gemäss Anspruch 1 enthält, wobei der rekombinante Vektor fähig ist, die für ein menschliches Interleukin-1α kodierende DNA in einem kompatiblen einzelligen Wirtsorganismus zu exprimieren.

3. Ein einzelliger Organismus, der einen rekombinanten Vektor gemäss Anspruch 2 enthält, wobei der einzellige Organismus fähig ist, die DNA, die ein menschliches Interleukin-1α Polypeptid kodiert, zu exprimieren.

4. Ein Verfahren zur Herstellung eines rekombinanten, menschlichen Interleukin-1α Polypeptides, wie es in Anspruch 1 definiert ist, dadurch gekennzeichnet, dass:
(a) ein einzelliger Organismus gemäss Anspruch 3 unter Bedingungen kultiviert wird, die für die Expression der DNA, die ein menschliches Interleukin-1α Polypeptid kodiert, geeignet sind; und
(b) das menschliche Interleukin-1α Polypeptid aus der Kultur isoliert und bis zur Homogenität gereinigt wird.

## Patentansprüche (Patentansprüche für folgende(n) Vertragsstaat(en): GR)

1. Ein Verfahren zur Herstellung eines homogenen rekombinanten, menschlichen Interleukin-1α Polypeptides mit der Aminosäuresequenz 
Ser Phe Leu Ser Asn Val Lys Tyr Asn Phe MET Arg Ile Ile Lys Tyr
Glu Phe Ile Leu Asn Asp Ala Leu Asn Gln Ser Ile Ile Arg Ala Asn
Asp Gln Tyr Leu Thr Ala Ala Ala Leu His Asn Leu Asp Glu Ala Val
Lys Phe Asp MET Gly Ala Tyr Lys Ser Ser Lys Asp Asp Ala Lys Ile
Thr Val Ile Leu Arg Ile Ser Lys Thr Gln Leu Tyr Val Thr Ala Gln
Asp Glu Asp Gln Pro Val Leu Leu Lys Glu MET Pro Glu Ile Pro Lys
Thr Ile Thr Gly Ser Glu Thr Asn Leu Leu Phe Phe Trp Glu Thr His
Gly Thr Lys Asn Tyr Phe Thr Ser Val Ala His Pro Asn Leu Phe Ile
Ala Thr Lys Gln Asp Tyr Trp Val Cys Leu Ala Gly Gly Pro Pro Ser
Ile Thr Asp Phe Gln Ile Leu Glu Asn Gln Ala.
 dadurch gekennzeichnet, dass
(a) ein einzelliger Organismus, der mit einem rekombinanten Vektor transformiert ist, der eine DNA enthält, die für das genannte Polypeptid kodiert, unter Bedingungen, die für die Expression der DNA geeignet sind, kultiviert wird; und
(b) das menschliche Interleukin-1α Polypeptid aus der Kultur isoliert und bis zur Homogenität gereinigt wird.

2. Ein Verfahren zur Herstellung eines einzelligen Organismus, der fähig ist, ein Polypeptid, wie es in Anspruch 1 definiert ist, zu exprimieren, dadurch gekennzeichnet, dass ein einzelliger Organismus auf bekannte Art und Weise mit einem rekombinanten Vektor, der eine DNA enthält, die für das genannte Polypeptid kodiert, transformiert wird.

3. Ein einzelliger Organismus, der einen rekombinanten Vektor enthält, der eine DNA enthält, die für ein menschliches Interleukin-1α, wie es in Anspruch 1 definiert ist, kodiert, wobei der einzellige Organismus fähig ist, die DNA zu exprimieren.

## Claims (Claims for the following Contracting State(s): AT, BE, CH, DE, FR, GB, IT, LI, LU, NL, SE)

1. A DNA coding for a human interleukin-la polypeptide having the amino acid sequence

Ser Phe Leu Ser Asn Val Lys Tyr Asn Phe MET Arg Ile Ile Lys Tyr
Glu Phe Ile Leu Asn Asp Ala Leu Asn Gln Ser Ile Ile Arg Ala Asn
Asp Gln Tyr Leu Thr Ala Ala Ala Leu His Asn Leu Asp Glu Ala Val
Lys Phe Asp MET Gly Ala Tyr Lys Ser Ser Lys Asp Asp Ala Lys Ile
Thr Val Ile Leu Arg Ile Ser Lys Thr Gln Leu Tyr Val Thr Ala Gln
Asp Glu Asp Gln Pro Val Leu Leu Lys Glu MET Pro Glu Ile Pro Lys
Thr Ile Thr Gly Ser Glu Thr Asn Leu Leu Phe Phe Trp Glu Thr His
Gly Thr Lys Asn Tyr Phe Thr Ser Val Ala His Pro Asn Leu Phe Ile
Ala Thr Lys Gln Asp Tyr Trp Val Cys Leu Ala Gly Gly Pro Pro Ser
Ile Thr Asp Phe Gln Ile Leu Glu Asn Gln Ala

2. A recombinant vector containing a DNA according to claim 1, which recombinant vector is capable of expressing the DNA coding for a human interleukin-1α in a compatible unicellular host organism.

3. A unicellular organism containing a recombinant vector according to claim 2, which unicellular organism is capable of expressing the DNA encoding the human interleukin-lapolypeptide.

4. A process for the preparation of a recombinant human interleukin-1α polypeptide as defined in claim 1, characterized by
(a) culturing a unicellular organism according to claim 3 under conditions suitable for the expression of the DNA encoding a human interleukin-1α polypeptide; and
(b) isolating the human interleukin-1α polypeptide from the culture and purifying it to homogeneity.

## Claims (Claims for the following Contracting State(s): GR)

1. A process for the preparation of a homogeneous recombinant human interleukin-1α polypeptide having the amino acid sequence 
Ser Phe Leu Ser Asn Val Lys Tyr Asn Phe MET Arg Ile Ile Lys Tyr
Glu Phe Ile Leu Asn Asp Ala Leu Asn Gln Ser Ile Ile Arg Ala Asn
Asp Gln Tyr Leu Thr Ala Ala Ala Leu His Asn Leu Asp Glu Ala Val
Lys Phe Asp MET Gly Ala Tyr Lys Ser Ser Lys Asp Asp Ala Lys Ile
Thr Val Ile Leu Arg Ile Ser Lys Thr Gln Leu Tyr Val Thr Ala Gln
Asp Glu Asp Gln Pro Val Leu Leu Lys Glu MET Pro Glu Ile Pro Lys
Thr Ile Thr Gly Ser Glu Thr Asn Leu Leu Phe Phe Trp Glu Thr His
Gly Thr Lys Asn Tyr Phe Thr Ser Val Ala His Pro Asn Leu Phe Ile
Ala Thr Lys Gln Asp Tyr Trp Val Cys Leu Ala Gly Gly Pro Pro Ser
Ile Thr Asp Phe Gln Ile Leu Glu Asn Gln Ala,
 characterized by
(a) culturing a unicellular organism transformed with a recombinant vector containing a DNA coding for said polypeptide under conditions suitable for the expression of the DNA; and
(b) isolating the human interleukin-1α polypeptide form the culture and purifying it to homogeneity.

2. A process for the preparation of a unicellular organism capable of expressing a polypeptide as defined in claim 1,
characterized by transforming a unicellular organism in a manner known per se with a recombinant vector containing a DNA coding for said polypeptide.

3. A unicellular organism containing a recombinant vector which contains a DNA coding for a human interleukin-1α as defined in claim 1, which unicellular organism is capable of expressing the DNA.

## Revendications (Revendications pour l'(les) Etat(s) contractant(s) suivant(s): AT, BE, CH, DE, FR, GB, IT, LI, LU, NL, SE)

1. ADN codant pour un polypeptide d'interleukine-1α humaine, comportant la séquence d'aminoacides

Ser Phe Leu Ser Asn Val Lys Tyr Asn Phe MET Arg Ile Ile Lys Tyr
Glu Phe Ile Leu Asn Asp Ala Leu Asn Gln Ser Ile Ile Arg Ala Asn
Asp Gln Tyr Leu Thr Ala Ala Ala Leu His Asn Leu Asp Glu Ala Val
Lys Phe Asp MET Gly Ala Tyr Lys Ser Ser Lys Asp Asp Ala Lys Ile
Thr Val Ile Leu Arg Ile Ser Lys Thr Gln Leu Tyr Val Thr Ala Gln
Asp Glu Asp Gln Pro Val Leu Leu Lys Glu MET Pro Glu Ile Pro Lys
Thr Ile Thr Gly Ser Glu Thr Asn Leu Leu Phe Phe Trp Glu Thr His
Gly Thr Lys Asn Tyr Phe Thr Ser Val Ala His Pro Asn Leu Phe Ile
Ala Thr Lys Gln Asp Tyr Trp Val Cys Leu Ala Gly Gly Pro Pro Ser
Ile Thr Asp Phe Gln Ile Leu Glu Asn Gln Ala

2. Vecteur recombinant qui contient un ADN selon la revendication 1, le vecteur recombinant étant capable d'exprimer, dans un organisme hôte monocellulaire compatible, l'ADN codant pour une interleukine-1α humaine.

3. Organisme monocellulaire qui contient un vecteur recombinant selon la revendication 2, l'organisme monocellulaire étant capable d'exprimer l'ADN qui code pour un polypeptide d'interleukine-1α humaine.

4. Procédé pour la production d'un polypeptide recombinant d'interleukine-1α humaine, tel qu'il est défini dans la revendication 1, caractérisé en ce que:
(a) on cultive un organisme monocellulaire selon la revendication 3, dans des conditions qui sont appropriées à l'expression de l'ADN qui code pour un polypeptide d'interleukine-1α humaine; et
(b) on isole à partir de la culture le polypeptide d'interleukine-1α humaine et on le purifie jusqu'à l'homogénéité.

## Revendications (Revendications pour l'(les) Etat(s) contractant(s) suivant(s): GR)

1. Procédé pour la production d'un polypeptide recombinant homogène d'interleukine-1α humaine, comportant la séquence d'aminoacides 
Ser Phe Leu Ser Asn Val Lys Tyr Asn Phe MET Arg Ile Ile Lys Tyr
Glu Phe Ile Leu Asn Asp Ala Leu Asn Gln Ser Ile Ile Arg Ala Asn
Asp Gln Tyr Leu Thr Ala Ala Ala Leu His Asn Leu Asp Glu Ala Val
Lys Phe Asp MET Gly Ala Tyr Lys Ser Ser Lys Asp Asp Ala Lys Ile
Thr Val Ile Leu Arg Ile Ser Lys Thr Gln Leu Tyr Val Thr Ala Gln
Asp Glu Asp Gln Pro Val Leu Leu Lys Glu MET Pro Glu Ile Pro Lys
Thr Ile Thr Gly Ser Glu Thr Asn Leu Leu Phe Phe Trp Glu Thr His
Gly Thr Lys Asn Tyr Phe Thr Ser Val Ala His Pro Asn Leu Phe Ile
Ala Thr Lys Gln Asp Tyr Trp Val Cys Leu Ala Gly Gly Pro Pro Ser
Ile Thr Asp Phe Gln Ile Leu Glu Asn Gln Ala,
 caractérisé en ce que:
(a) on cultive un organisme monocellulaire qui est transformé par un vecteur recombinant qui contient un ADN codant pour ledit polypeptide, dans des conditions qui sont appropriées à l'expression de l'ADN; et
(b) on isole à partir de la culture le polypeptide d'interleukine-1α humaine et on le purifie jusqu'à l'homogénéité.

2. Procédé pour la production d'un organisme monocellulaire qui est capable d'exprimer un polypeptide, tel qu'il est défini dans la revendication 1, caractérisé en ce qu'un organisme monocellulaire est transformé de façon connue par un vecteur recombinant qui contient un ADN qui code pour ledit polypeptide.

3. Organisme monocellulaire qui contient un vecteur recombinant qui contient un ADN codant pour une interleukine-1α humaine, telle qu'elle est définie dans la revendication 1, l'organisme monocellulaire étant capable d'exprimer l'ADN.
